# EUROPEAN PATENT SPECIFICATION

(11) **EP 3 131 639 B1**
(45) Date of publication and mention of the grant of the patent: **26.09.2018**
(21) Application number: 15715282.8
(22) Date of filing: 13.04.2015
(51) Int. Cl.: A61Q 11/00, A61K 8/81, A61K 8/02, A61K 8/19

(54) **SOLID ORAL CARE COMPOSITIONS COMPRISING CALCIUM CARBONATE AND POLYETHYLENE OXIDE**
FESTE MUNDPFLEGEZUSAMMENSETZUNGEN ENTHALTEND KALZIUMCARBONAT UND POLYETHYLENOXID
COMPOSITIONS DE SOIN ORAL SOLIDE COMPRENANT DU CARBONATE DE CALCIUM ET DE L'OXYDE DE POLYÉTHYLÈNE

(30) Priority: 17.04.2014 EP 14165046
(43) Date of publication of application: 22.02.2017
(73) Proprietor: Unilever PLC, London Greater London EC4Y 0DY (GB); Unilever N.V., 3013 AL Rotterdam (NL)
(72) Inventor: ASHCROFT, Alexander Thomas, Wirral Merseyside CH63 3JW (GB); WILSON, William John, Wirral Merseyside CH63 3JW (GB)
(74) Representative: Tansley, Sally Elizabeth
(86) International application number: PCT/EP2015/057920
(87) International publication number: WO 2015/158637

(56) References cited:
- EP-A2- 1 566 160
- WO-A1-2007/091856
- WO-A1-2013/077127
- JP-A- 2011 126 819
- KR-A- 20090 076 440
- DATABASE GNPD [Online] MINTEL; June 2000 (2000-06), Warner-Lambert: "Extra Strength Freshmint", XP002730681, Database accession no. 10068164

## Description

### Field of the Invention

The present invention is concerned with solid oral care compositions.

### Background of the Invention

Oral care compositions such as dentifrices typically contain dentally acceptable abrasive, humectant, water, and water-soluble polymer which serves as a thickener and binder for the ingredients. A variety of other ingredients such as flavours, sweeteners, preservatives and fluoride are also utilized at low levels.

Efforts have been made in the prior art to provide anhydrous or reduced water content dentifrice formulations, as a more suitable vehicle for water-incompatible oral care actives. Examples include solid product forms such as powders or tablets.

US 3,431,339 describes a compressed dental tablet for use in place of toothpaste. The tablet contains a blend of a water-soluble anticaries agent, polishing agent, foaming agent and releasable matrix, all in a substantially anhydrous form. Advantages of the format are stated to be improved storage stability of the anticaries agent in the dry tablet and improved cleaning of the lingual surfaces, compared to conventional toothpaste.

US 3,116,208 discloses a dental cleanser in tablet form comprised of calcium carbonate and sodium lauryl sulphate. The tablet may be crushed by the teeth and the sodium lauryl sulphate causes foaming upon brushing the teeth.

WO88/10110 describes a tooth cleaning tablet designed to form a self-foaming paste when chewed in the mouth. The tablets contain "polishing agent, swelling agent, foaming agent, filling agent, taste-giving agent, wetting agent, lubricative agent, glidant, and tooth protecting agent, preferably stannous fluoride". The tablets are substantially water free to prevent degradation of the stannous fluoride.

US2007/0154409 describes a tooth cleansing tablet consisting of a quantity of tooth powder formed into a firm tablet. The tablet is made from a combination of a mild tooth abrasive with a binding agent and flavouring. The tablet is protected by a coating which provides water resistance and can be made of any ingredient commonly used to form the coatings on candies or gum balls.

US 5,804,165 discloses an antiplaque oral composition using a source of carbon dioxide, an insoluble silica abrasive and xylitol. The carbon dioxide comes from a bicarbonate. The effervescent tablet converts to a solid silica containing suspension in the saliva of the oral cavity.

US 8,192,724 describes a water soluble, non-abrasive, effervescent, oral care tablet for use in the oral cavity. The tablet includes a carbon dioxide source, an acid source and a tablet forming material admixed to form a chewable tablet having less than 0.2% water.

WO 2007/091856 discloses oral care tablets and aims at preparations with an improved mouth feel. Despite the above prior art, solid product forms such as powders or tablets have not gained widespread acceptance amongst consumers. Disadvantages include undesirable sensory characteristics such as gritty or chalky mouth feel.

It is an object of the present invention to provide tablets with improved mouth feel.

### Summary of the Invention

The present invention provides a solid oral care tablet composition for use in the cleaning of teeth, the composition comprising calcium carbonate and polyethylene oxide.

The scope of the invention is defined by the appended claims. The invention also relates to a the use of the tablets in method of cleaning the teeth comprising the step of chewing a tablet as described above.

A further aspect of the invention is the use of polyethylene oxide to impart a smooth feeling to a tablet described above.

### Detailed Description of the Invention

The present invention relates to solid oral care composition, preferably the composition is in the form of a tablet, more preferably in the form a compressed tablet.

Composition of the invention comprise calcium carbonate, the calcium carbonate being a particulate material which acts to mechanically remove debris from a tooth surface and debride plaque.

Suitable particulate calcium carbonates may be characterised by the specific shape and size of their constituent primary particles.

By "primary particles" is meant individual particles, defined as the smallest discrete particles that can be seen by electron microscopy analysis (such as, for example, individual crystals).

Polymeric molecular weights described in the context of the present invention relate to Mw.

The primary particles may associate under certain conditions to form larger secondary structures such as aggregates or agglomerates.

For the purposes of the present invention, a suitable source of particulate calcium carbonate includes crystalline calcium carbonates.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline calcium carbonates suitable for use in the invention are available naturally (through the extraction and processing of natural ores) or synthetically (through chemical precipitation). There are three main crystalline polymorphs: calcite, aragonite, and less commonly found, vaterite. The vaterite form of calcium carbonate is metastable and irreversibly transforms into calcite and aragonite. There are many different morphologies(crystal habits) for each of these crystalline forms. The calcite crystalline polymorph is the most commonly used crystal form of calcium carbonate. Over 300 crystalline forms of calcite have been reported in the literature.

Particulate calcium carbonates suitable for use in the invention (such as the materials described above) have an average particle size which can vary over a wide range. Usually the average particle size is 50 microns or less.

Particle (e.g. crystal) size may be determined by standard techniques known to those skilled in the art such as sedimentation. For the present invention the particle size should be determined using a Malvern mastersizer. In the context of the present application particle size measurements relate to d(50).

Good cleaning performance has been obtained with crystalline calcium carbonates in which at least 50% by weight, preferably at least 80% by weight of the crystals are rhombohedral calcite crystals with an average particle size d(50) of 30 microns or less, preferably15 microns or less. Most preferable are crystalline calcium carbonates with an average particle size d(50)from 0.1 to 30 microns, preferably 0.1 to 15 microns, most preferably 2 to 10 microns (measured using a Malvern mastersizer).

Suitable sources of crystalline calcium carbonate of the size and shape defined above include natural ground calcium carbonate abrasives, generally obtainable from mining and mechanical grinding of sedimentary rocks such as limestone or chalk, or metamorphic rocks such as marble. Natural calcium carbonate is usually of the calcitic polymorph, with a crystal morphology which may typically be characterised as rhombohedral.

Preferred natural ground calcium carbonate abrasives of the type defined above are selected from ground limestone, chalk or marble, optionally refined or partially refined to remove impurities, and having an average particle size d(50)("median ESD" as described above) of about 30 microns or less, more preferably ranging from about 1 to 15 microns, and most preferably ranging from about 2 to 10 microns. Commercially available examples include those materials sold by the company OMYA™ AG under the names OMYACARB™ 2-AV and OMYACARB™ 5-AV. These are fine ground high purity white marble abrasives having an average particle size ("median ESD" as described above) of about 2 and about 5 microns respectively.

Other types of particulate calcium carbonate may also be suitable, depending on the particular balance of cleaning and abrasion required from a consumer perspective. Mixtures of any of the above described abrasive cleaning agents may also be used.

The total amount of calcium carbonate in the composition of the invention will depend on the particular agent (or agents) used, but generally ranges from 3 to 80%, preferably from 15% to 75%, more preferably from 30% to 70% most preferably form 40 to 50 wt% by weight by total weight of the composition.

Compositions of the invention comprise polyethylene oxide that has a molecular weight ranging greater than 500,000 (Mw), more preferably ranging from 1000,000 to 7000,000 (Mw).

The weight of polyethylene oxide in the composition is preferably from 0.1 to 3 wt%, more preferably from 0.5 to 2 wt% of the total composition.

It is preferable if the polyethylene oxide is present as part of a mixture within the tablet rather than an external coating.

Preferably the weight ratio of polyethylene oxide to calcium carbonate is from 1: to 1:10 to 1:100, more preferably from 1:25 to 1:55.

Suitable foaming agents for use in forming the composition include surfactants. Surfactants help to increase the rate of dissolution of the solid oral care composition when in contact with saliva, and assist in foaming of the composition during usage.

Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene oxide, ethoxylates of fatty acid monoglycerides and diglycerides, and ethylene oxide/propylene oxide block polymers. Other suitable surfactants include amphoteric surfactants, such as betaines or sulphobetaines.

Preferred surfactants for use in the invention include those anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

Mixtures of any of the above described materials may also be used.

The total amount of surfactant incorporated into the composition of the invention generally ranges from about 0.2 to about 5%, by total weight surfactant based on the total weight of the composition.

The composition may also include various additional ingredients to improve aspects such as ease of processing, product performance and/or consumer acceptability.

For example, the composition may include effervescent agents which promote foaming by the provision of bubbles. Suitable effervescent agents for use in this context include acid/carbonate salt couples which provide effervescence by the reaction of the carbonate salt with the acid. Sodium bicarbonate and sodium carbonate represent preferred carbonate salts. However potassium, ammonium, magnesium, calcium carbonate or other metal or organic salts can also be used. Suitable acids include citric acid, fumaric acid, tartaric acid, malic acid, adipic acid and other edible acids.

The amount of effervescent agent incorporated into the composition of the composition of the invention generally ranges from about 0.5 to about 10%, preferably from about 1 to 7%, by total weight effervescent agent (e.g. acid/carbonate salt couple) based on the total weight of the composition.

Mixtures of any of the above described materials may also be used.

The composition will preferably include one or more organic polyols having 2 or more hydroxyl groups in the molecule (hereinafter termed "organic polyols"). These materials may serve as binders to aid in compression of the composition during processing. They may also act as humectants. Humectants help to keep the composition from hardening or crystallizing upon exposure to air. They also help to give the composition a moist feel to the mouth, and may in some cases impart a desirable sweetness. Suitable organic polyols for use in this context include sucrose, dextrose, maltose, fructose, glycerol, sorbitol, xylitol, mannitol, lactitol, maltitol, isomalt, erythritol, polyethylene oxide, polypropylene glycol, propylene glycol, and hydrogenated partially hydrolyzed polysaccharides such as hydrogenated starch hydrolysates. Preferred organic polyols for use in this context are non-cariogenic polyhydric alcohols such as glycerol, sorbitol, maltitol and xylitol.

Mixtures of any of the above described materials may also be used.

The amount of organic polyol incorporated into the composition of the composition of the invention generally ranges from about 5 to about 70%, preferably from about 10 to 40%, by total weight organic polyol based on the total weight of the composition.

The composition of the composition of the invention is typically non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% by weight based on the total weight of the composition.

The solid oral care composition of the invention will generally take the form of a discrete, single unit dose such as a pellet, pastille or tablet. Such a single unit dose will typically range in size from 200 mg to 5000 mg, preferably from 250 mg to 2000 mg, more preferably from 500 to 1500 mg.

Compositions according to the invention are particularly suitable as a vehicle for oral care actives which may be physically or chemically incompatible with water, or which may function less efficiently in an aqueous environment.

Specific examples of oral care actives which may be particularly suitable for inclusion in the compositions of the invention are:
water-soluble or sparingly water-soluble sources of zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate;
oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8-hydroxyquinoline derivatives), lactoperoxidase, lactoferrin, lysozyme and mixtures thereof;
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.
agents for the remineralisation of teeth (i.e the *in situ* generation of hydroxyapatite on teeth), such as a mixture of a calcium source and a phosphate source which, when delivered to the teeth results in the *in situ* generation of hydroxyapatite on teeth.

Mixtures of any of the above described materials may also be used.

Compositions of the present invention may also contain further optional ingredients customary in the art such as anticalculus agents, buffers, flavouring agents, sweetening agents, colouring agents, opacifying agents, preservatives, antisensitivity agents, antimicrobial agents and the like.

The solid oral care composition of the invention is used to clean the surfaces of the oral cavity. The composition is an oral composition that is not intentionally swallowed for purposes of systemic administration of therapeutic agents, but is applied to the oral cavity, used to treat the oral cavity and then expectorated. Typically the composition is used in conjunction with a cleaning implement such as a toothbrush, usually by applying it to the bristles of the toothbrush and then brushing the accessible surfaces of the oral cavity.

Tablets of the invention are compressed and are preferably manufactured using a compaction process to 1 to 8 tons. Preferably the tablets are not coated.

The invention is further illustrated with reference to the following, non-limiting Examples. Examples of the invention are illustrated by a number, comparative Examples are illustrated by a letter.

### EXAMPLE

| Tradename | Chemical Name | Example A | Example 1 |
|---|---|---|---|
| | | Wt% | Wt% |
| Empicol LZN | Sodium lauryl sulphate | 3 | 3 |
| Menthol | Menthol | 0.5 | 0.5 |
| Novamint | Synthetic flavour | 6 | 6 |
| Chalk | Calcium carbonate | 45.2 | 45.2 |
| Sodium saccharin | Sodium saccharin | 0.3 | 0.3 |
| Xylisorb DC | Xylitol | To 100 | To 100 |
| Polyox WSR-301 | Poly(ethylene) oxide polymer | 0 | 1 |

To form the tablet the materials were blended together. Once blended a 1g amount is weighed into a tablet die and compressed to the desired force (3 ton).

The tablet was assessed by a trained panel. All tablets were presented in petri dishes. The tablets were placed in the mouth and chewed, then when foam developed; panellists brushed their teeth and eventually spit out any residue then rinsed if needed. The panel 'rated' the intensity of the smooth feeling attribute.

In-between prototypes participants were asked to eat 2-3 crisps (Walker ready salted) and rinse their mouth with room-temperature water. This process cleanses the palate of previous tastes. After the last prototype, only rinsing if required.

The results of the panel test were as follows:

| Sensory Attribute | Score Example 1 | Score Example A |
|---|---|---|
| Smooth feel in mouth | 70.4 | 61.9 |

The higher the score the more intense the attribute.

The results show that tablets of the invention feel less chalky in the mouth after use and fell smooth in the mouth during use.

## Claims

1. A solid oral care composition in the form of a compressed tablet for use in the cleaning of teeth, the composition comprising calcium carbonate and polyethylene oxide, in which the polyethylene oxide has a molecular weight ranging greater than 500,000 (Mw).

2. A solid oral care composition according to claim 1 in which the polyethylene oxide has a molecular weight ranging from 1000,000 to 7000,000 (Mw).

3. A solid oral care composition according to any preceding claim in which the level of polyethylene oxide is from 0.1 to 3 wt% of the total composition.

4. A solid oral care composition according to any preceding claim in which the calcium carbonate has a particle size ranging from 0.1 to 30 microns, preferably 0.1 to 15 microns, most preferably 2 to 10 microns as measured by Malvern mastersizer.

5. A solid oral care composition according to any preceding claim in which the weight ratio of polyethylene oxide to calcium carbonate is from 1:30 to 1:50.

6. A solid oral care composition according to any preceding claim which further comprises a foaming agent selected from the group consisting of anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

7. A solid oral care composition according to any preceding claim, in which the composition also includes one or more organic polyols selected from glycerol, sorbitol, maltitol, xylitol and mixtures thereof.

8. A solid oral care composition according to any preceding claim in which the polyethylene oxide is present as part of a mixture within the solid oral care composition.

9. A tablet as described in any previous claim for use in the cleaning of teeth comprising chewing the tablet.

10. Use of polyethylene oxide to impart a smooth feeling to a compressed tablet described in any previous claim.

## Patentansprüche

1. Feste Mundpflegezusammensetzung in Form einer Presstablette zur Verwendung beim Reinigen von Zähnen, wobei die Zusammensetzung Calciumcarbonat und Polyethylenoxid umfasst, in welcher das Polyethylenoxid ein Molekulargewicht von mehr als 500.000 (Mw) aufweist.

2. Feste Mundpflegezusammensetzung nach Anspruch 1, in welcher das Polyethylenoxid ein Molekulargewicht in dem Bereich von 1.000.000 bis 7.000.000 (Mw) aufweist.

3. Feste Mundpflegezusammensetzung nach irgendeinem vorhergehenden Anspruch, in welcher die Konzentration des Polyethylenoxids von 0,1 bis 3 Gew.-% der gesamten Zusammensetzung beträgt.

4. Feste Mundpflegezusammensetzung nach irgendeinem vorhergehenden Anspruch, in welcher das Calciumcarbonat eine Partikelgröße in dem Bereich von 0,1 bis 30 Mikron, vorzugsweise von 0,1 bis 15 Mikron, höchst bevorzugt von 2 bis 10 Mikron, gemessen mittels eines Malvern-Mastersizers, aufweist.

5. Feste Mundpflegezusammensetzung nach irgendeinem vorhergehenden Anspruch, in welcher das Gewichtsverhältnis von Polyethylenoxid zu Calciumcarbonat von 1:30 bis 1:50 beträgt.

6. Feste Mundpflegezusammensetzung nach irgendeinem vorhergehenden Anspruch, welche ferner ein Schaumbildungsmittel enthält, das aus der Gruppe ausgewählt ist, die aus wasserfreien Tensiden besteht, die aus Natriumlaurylsulfat, Natriumlaurylsulfoacetat, Cocamidopropylbetain, Natriumalphaolefinsulfonat, Dioctylnatriumsulfosuccinat, Natriumdodecylbenzolsulfonat und Mischungen davon ausgewählt sind.

7. Feste Mundpflegezusammensetzung nach irgendeinem vorhergehenden Anspruch, in welcher die Zusammensetzung ebenfalls ein oder mehrere organische Polyole enthält, ausgewählt aus Glycerin, Sorbit, Maltit, Xylit und Mischungen davon.

8. Feste Mundpflegezusammensetzung nach irgendeinem vorhergehenden Anspruch, in welcher das Polyethylenoxid als Teil einer Mischung innerhalb der festen Mundpflegezusammensetzung vorliegt.

9. Tablette, wie in irgendeinem vorhergehenden Anspruch beschrieben, zur Verwendung bei der Reinigung von Zähnen, welche das Kauen der Tablette umfasst.

10. Verwendung von Polyethylenoxid, um einer Presstablette, beschrieben in irgendeinem vorhergehenden Anspruch, ein geschmeidiges Gefühl zu verleihen.

## Revendications

1. Composition solide pour le soin oral dans la forme d'un comprimé formé par compression destiné à une utilisation dans le nettoyage de dents, la composition comprenant du carbonate de calcium et du poly(oxyde d'éthylène), dans laquelle le poly(oxyde d'éthylène) présente une masse moléculaire supérieure à 500 000 (Mw).

2. Composition solide pour le soin oral selon la revendication 1, dans laquelle le poly(oxyde d'éthylène) présente une masse moléculaire de 1 000 000 à 7 000 000 (Mw).

3. Composition solide pour le soin oral selon l'une quelconque des revendications précédentes, dans laquelle la teneur en poly(oxyde d'éthylène) est de 0,1 à 3 % en masse de la composition totale.

4. Composition solide pour le soin oral selon l'une quelconque des revendications précédentes, dans laquelle le carbonate de calcium présente une taille de particule de 0,1 à 30 microns, de préférence de 0,1 à 15 microns, encore mieux de 2 à 10 microns comme mesurée par un Mastersizer Malvern.

5. Composition solide pour le soin oral selon l'une quelconque des revendications précédentes, dans laquelle le rapport massique de poly(oxyde d'éthylène) à carbonate de calcium est de 1:30 à 1:50.

6. Composition solide pour le soin oral selon l'une quelconque des revendications précédentes qui comprend de plus un agent moussant choisi dans le groupe constitué de tensioactifs anhydres choisis parmi le laurylsulfate de sodium, laurylsulfoacétate de sodium, cocamidopropylbétaïne, oléfine-alpha-sulfonate de sodium, sulfosuccinate de dioctylsodium, dodécylbenzènesulfonate de sodium et mélanges de ceux-ci.

7. Composition solide pour le soin oral selon l'une quelconque des revendications précédentes, dans laquelle la composition comprend également un ou plusieurs polyols organiques choisis parmi le glycérol, sorbitol, maltitol, xylitol et mélanges de ceux-ci.

8. Composition solide pour le soin oral selon l'une quelconque des revendications précédentes, dans laquelle le poly(oxyde d'éthylène) est présent comme une partie d'un mélange dans la composition solide pour le soin oral.

9. Comprimé comme décrit dans l'une quelconque des revendications précédentes destiné à une utilisation dans le nettoyage de dents comprenant le masticage du comprimé.

10. Utilisation de poly(oxyde d'éthylène) pour communiquer une sensation lisse à un comprimé formé par compression décrit dans l'une quelconque des revendications précédentes.
